# EUROPEAN PATENT APPLICATION

(11) **EP 2 647 636 A1**
(43) Date of publication of application: **09.10.2013**
(21) Application number: 11845985.8
(22) Date of filing: 30.11.2011
(51) Int. Cl.: C07D 413/14

(54) **METHOD FOR PRODUCING MALEATE USING WET CRYSTAL**

(30) Priority: 30.11.2010 JP 2010266686
(71) Applicant: Kyorin Pharmaceutical Co., Ltd., Tokyo 101-8311 (JP)
(72) Inventor: TSUBUKI, Takeshi, Shimotsuga-gun Tochigi 329-0114 (JP)
(74) Representative: Vossius & Partner
(86) International application number: PCT/JP2011/006704
(87) International publication number: WO 2012/073508

(57) **Abstract**

[Problem] To efficiently (without carrying out column purification) provide a high-purity maleate having excellent storage stability, solubility, crystallinity and ease of handling according to the present invention.

[Solution] This method for producing the maleate represented by formula (1) includes obtaining the compound represented by formula (2) by reacting the compound represented by formula (3) with the compound represented by formula (4) in the presence of a salt, precipitating crystals of the compound represented by formula (2) by mixing the obtained compound represented by formula (2) with acetone and water, and obtaining the maleate represented by formula (1) by adding maleic acid to an acetone solution of the compound represented by formula (2), prepared by dissolving the crystals of the obtained compound represented by formula (2) in acetone.

## Description

### Field

The present invention relates to a method for producing a maleate using a wet crystal of a benz[d][1,3]oxazine derivative.

### Background

Human neutrophil elastase is a kind of serine hydrolase released from granules of neutrophil, which appear during infection or inflammatory disease. Neutrophil elastase is an enzyme hydrolyzing elastin, collagen, proteoglycan, fibronectin, and other proteins which constitute the interstitium of intravital connective tissues such as lung, cartilage, vascular wall, and skin. In addition, it has been clarified that neutrophil elastase acts on other proteins or cells.

Serine hydrolase including neutrophil elastase maintains homeostasis in the living body. The activity of serine hydrolase is controlled by endogenous protein inhibitor such as α1-protease inhibitor, α2-macrogloblin, and secretory leukocyte protease inhibitor. However, when a balance between neutrophil elastase and endogenous inhibitor is lost by excessive release of neutrophil elastase in the inflammation site or by decline in an inhibitor level, the control of neutrophil elastase activity cannot be maintained, and tissues are injured.

Examples of diseases involved in serine hydrolase include pulmonary emphysema, acute respiratory distress syndrome, adult respiratory distress syndrome (ARDS), idiopathic interstitial pneumonia (IIP), cystic pulmonary fibrosis, chronic interstitial pneumonia, chronic bronchitis, chronic sinopulmonary infection, diffuse panbronchiolitis, bronchiectasis, asthma, pancreatitis, nephritis, hepatic failure, rheumatoid arthritis, joint scleroma, osteoarthritis, psoriasis, periodontitis, atherosclerosis, rejection against organ transplant, premature amniorrhexis, bullous dermatosis, shock, sepsis, systemic lupus erythematosus (SLE), Crohn's disease, disseminated intracapillary coagulation (DIC), tissue injury after ischemia-reperfusion, formation of cornea cicatricial tissue, myelitis and the like. As a therapeutic agent for these diseases, the development of serine hydrolase inhibitor is expected.
A benz [d] [1, 3] oxazine derivatives have been reported as an excellent serine hydrolase inhibitor (Patent Literature 1).

### Citation List

### Patent Literature

Patent Literature 1: WO 2008/036379

### Summary of Invention

### Technical Problem

The benz[d][1,3]oxazine derivative described in Patent Literature 1 shows an excellent serine hydrolase inhibitory activity, but has low long-term storage stability. Therefore, a compound having more excellent physical properties is expected from the viewpoint of production of pharmaceuticals as an active pharmaceutical ingredient for manufacture. The present inventors have found a maleate of a benz[d] [1,3]oxazine derivative as a compound having excellent stability as an active pharmaceutical ingredient, solubility, and crystallinity (PCT/JP2010/005786).
The maleate can be produced through a free form (compound (2)). However, since the obtained free form is an amorphous solid and column purification and concentration of free form solution after the column purification are essential, this production is not preferable as an industrial production method. Further, since a concentrate of free form solution is a glassy solid, there are various problems (for example., production cost, re-purification, storage, transportation, and weighing) from the viewpoint of production.

### Solution to Problem

The present inventor has intensively investigated to establish an industrial method for producing a maleate of a benz[d] [1,3]oxazine derivative. As a result, the inventor has found that a free form of a benz [d] [1,3]oxazine derivative is crystallized in acetone and water and obtained as a wet crystal. And the inventor has also found that a maleate of a benz[d][1,3]oxazine derivative is crystallized from a wet crystal of a free form by a simple operation. Thus, the present invention has been completed.

The summary of the present invention is as follows:
1) A method for producing a crystal of a maleate represented by the formula (1), comprising
   reacting a compound represented by the formula (3) with a compound represented by the formula (4) in the presence of a base to obtain a compound represented by the formula (2);
   mixing the obtained compound represented by the formula
(2) with acetone and water to precipitate a crystal of the compound represented by the formula (2); and
   adding maleic acid to a solution of the compound represented by the formula (2) prepared by dissolving, in a solvent, the obtained crystal of the compound represented by the formula (2) directly or after being dried to obtain a crystal of a maleate represented by the formula (1).

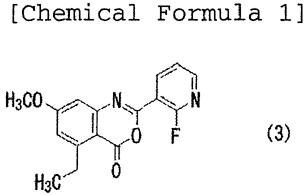

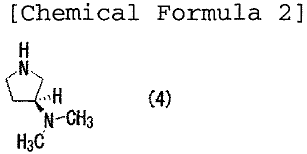

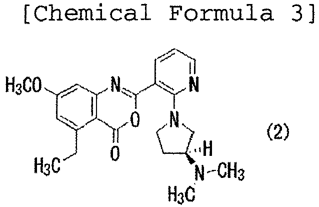

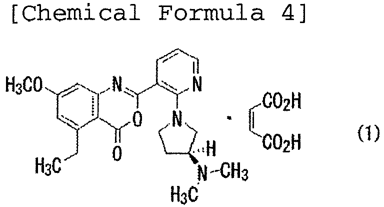

2) The method for producing a crystal of a maleate represented by the formula (1) according to the method of 1), wherein the crystal of the compound represented by the formula (2) is dried and then dissolved in the solvent to prepare the solution of the compound represented by the formula (2), and
the crystal of the compound represented by the formula (2) is dried under reduced pressure, or dried by dissolving the crystal in a solvent capable of separating from water into two layers, removing separated water, and concentrating the solution.

3) A crystal of a compound represented by the formula (2) having a particular peak at a diffraction angle (2θ ± 0.5°) of 15.4° in a powder X-ray diffraction.

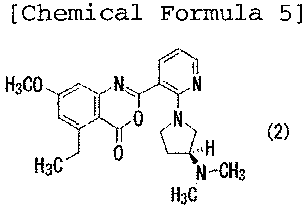

4) A crystal obtained by mixing a compound represented by the formula (2) with acetone and water to precipitate the crystal.

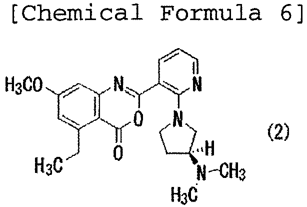

5) A method for producing a crystal of a maleate represented by the formula (1), comprising
dissolving the crystal described in 3) or 4) directly or after being dried in a solvent to prepare a solution of a compound represented by the formula (2); and
adding maleic acid to the obtained solution of the compound represented by the formula (2) to obtain a crystal of a maleate represented by the formula (1).

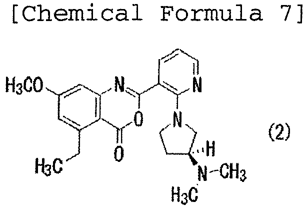

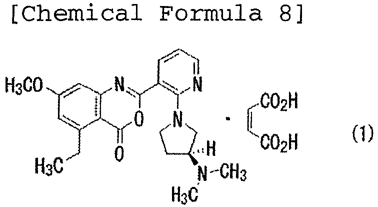

6) The method for producing a crystal of a maleate represented by the formula (1) according to the method of 5), wherein the crystal of the compound represented by the formula (2) is dried and then dissolved in the solvent to prepare the solution of the compound represented by the formula (2), and
the crystal of the compound represented by the formula (2) is dried under reduced pressure or dried by dissolving the crystal in a solvent capable of separating from water into two layers, removing separated water, and concentrating the solution.

### Advantageous Effects of Invention

The present invention can provide a compound represented by the formula (1), which is an active pharmaceutical ingredient having excellent stability, solubility, and crystallinity, at high purity and efficiency (without column purification).

### Brief Description of Drawings

FIG. 1 is a powder x-ray diffraction pattern of a crystal of a compound (2) (Test Example 1). The longitudinal axis indicates an intensity (cps), and the horizontal axis indicates a diffraction angle (2θ)[°].
FIG. 2 is a powder x-ray diffraction pattern of a dried product of the compound (2) in an amorphous form (Test Example 2). The longitudinal axis indicates an intensity (cps), and the horizontal axis indicates a diffraction angle (2θ)[°].
FIG. 3 is a powder x-ray diffraction pattern of a crystal of the compound (2) (Test Example 3). The longitudinal axis indicates an intensity (cps), and the horizontal axis indicates a diffraction angle (2θ)[°].
FIG. 4 is a powder x-ray diffraction pattern of a dried product of the compound (2) in an amorphous form (Test Example 3). The longitudinal axis indicates an intensity (cps), and the horizontal axis indicates a diffraction angle (2θ)[°].

### Description of Embodiments

The present invention relates to a method for producing a crystal of a maleate represented by the formula (1) (hereinafter also referred to as compound (1)) through a crystal of a compound represented by the formula (2) (hereinafter also referred to as compound (2)).

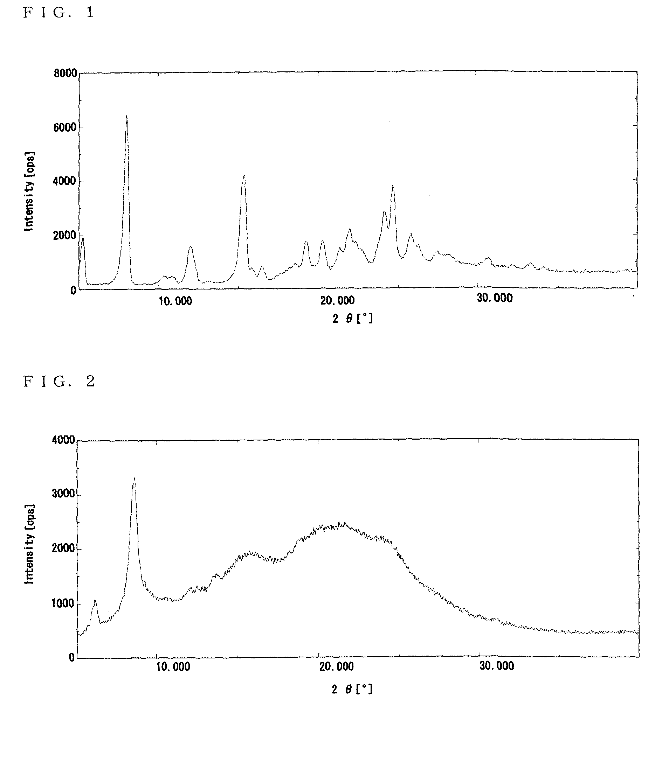

The compound (1) as a target compound of the production method of the present invention is 2-[2-((S)-3-dimethylaminopyrrolidin-1-yl)pyridin-3-yl]-5-et hyl-7-methoxy-4H-benz[d][1,3]oxazin-4-one maleate which has excellent solubility, stability, and crystallinity under certain conditions. The compound (1) also has excellent handleability since the compound (1) does not have adhesion water and the weight is not changed due to moisture absorption or moisture release.
On the other hand, since the compound (2) which is a production intermediate of the compound (1) has been obtained as a glassy solid, column purification for the compound (2) has been necessary. However, the present inventor has found that the compound (2) is crystallized under specific conditions. The crystal of the compound (2) found by the inventor is measured with copper radiation to obtain a powder x-ray diffraction pattern having peaks at diffraction angles (2θ ± 0.5°) of 8.02°, 12.0°, 15.4°, 24.2°, and 24.7°, like a powder x-ray diffraction pattern in FIG. 1 as an example. In particular, the crystal has a characteristic powder x-ray diffraction peak which is distinguishable from another crystal at a diffraction angle (2θ ± 0.5°) of 15.4°. The crystal of the compound (2) has physical properties which are excellent in the state, properties, and stability of the crystal as compared with an amorphous form.

The compound (2) can be obtained by a reaction of a compound represented by the formula (3) (hereinafter also referred to as compound (3)) with a compound represented by the formula (4) (hereinafter also referred to as compound (4)) in the presence of a base. A base used in the reaction is not particularly limited, but a lower alkylamine such as triethylamine is preferable. A solvent to be used is not particularly limited as long as it is not involved in the reaction. Examples of the solvent include a hydrophilic solvent such as dimethylformamide. The amount of the solvent to be used is not particularly limited. The amount of the solvent is preferably 5 to 15 times, and more preferably 7 times the amount of the compound represented by the formula (3) to smoothly promote the reaction. Although it is not particularly limited, in the reaction, to smoothly promote the reaction, it is preferable that the reaction is performed at 10 to 40°C, and more preferably at 30°C. In the specification, for example, the use of the solvent in an amount 10 times the amount of the compound means that 10 mL of solvent is used per one gram of the compound.

The compound (2) produced by the above method is mixed with acetone and water to precipitate a crystal of the compound (2).
For example, the mixing can be achieved by mixing a reaction solution containing the compound (2) produced by a reaction of the compound (3) with the compound (4) with acetone and water, or mixing an acetone solution of the product obtained by column purification of the compound (2) described in WO2008/036379 in water. At this time, acetone can be used in an amount 5 to 15 times, and preferably 9 times, the amount of the compound represented by the formula (3). Water for crystallization can be used in an amount 15 to 35 times, and preferably 25 times, the amount of the compound represented by the formula (3).
Acetone and water can be mixed with the reaction solution at 0 to 30°C, and preferably at 10°C.
The obtained crystal of the compound (2) can be used in a reaction to produce the compound (1) as a next reaction. The obtained crystal of the compound (2) is separated from the reaction solution which was mixed with acetone and water, for example, by filtration. After that, the crystal can be used in the next reaction as it is (directly) or after being dried. The crystal of the compound (2) may be stored under a wet condition. After the storage, the crystal of the compound (2) can be used in the next reaction directly or after being dried.
In the specification, the expression "directly" means that the crystal of the compound (2) is not subjected to a drying process.
In order to stably store the wet crystal of the compound (2), the wet crystal preferably has water in a content of 30 to 70 w/w%, and more preferably 40 to 60 w/w%.
The obtained crystal can be recrystallized in acetone and water to obtain a crystal at a higher chemical purity. Acetone used in the recrystallization can be used in an amount 10 to 20 times, and preferably 15 times, the amount of the dried compound (2). Water for the crystallization can be used in an amount 20 to 40 times, and preferably 30 times, the amount of the dried compound (2).

The compound (1) can be produced by using the compound (2), maleic acid, and a solvent capable of dissolving the compound (2). Specifically, maleic acid is added to the solution of the compound (2) to produce the compound (1). The solution of the compound (2) used herein can be prepared by dissolving the above crystal of the compound (2) obtained by mixing the compound (2) with acetone and water in the solvent directly or after being dried. In particular, it is preferable that the crystal of the compound (2) be dissolved in the solvent after being dried. The drying used herein includes not only drying under reduced pressure but also drying by dissolving the crystal of the compound (2) obtained by mixing the compound (2) with acetone and water in a solvent capable of separating from water into two layers (for example, ethyl acetate, toluene, methylene chloride, and chloroform), removing separated water, and concentrating the solution. In the concentration after removal of separated water, the solution may be concentrated after being dried using a drying agent such as anhydrous sodium sulfate, or as it is without using a drying agent.
The solvent used in the production is preferably an aprotic organic solvent such as acetonitrile, ethyl acetate, acetone, or tetrahydrofuran, and particularly preferably acetone. A diluted solvent may be further added to a suspension of crystalline solid in which the compound (1) is precipitated as a crystal. The diluted solvent is preferably ethyl acetate, tert-butyl methyl ether, tetrahydrofuran, diethyl ether, or diisopropyl ether, and particularly preferably ethyl acetate, tert-butyl methyl ether, or diisopropyl ether.

The obtained compound (1) can be further recrystallized by an appropriate solvent. As the solvent used in the recrystallization, acetone, tetrahydrofuran, acetonitrile, 1,2-dimethoxyethane, ethyl acetate, or water can be used alone or in combination. A mixed solvent of acetone and water is preferable. Examples of the diluted solvent added to precipitate a crystal include diisopropyl ether, ethyl acetate, and tert-butyl methyl ether.

Hereinafter, the present invention will be described in detail by Examples and Test Examples, and the present invention is not limited to these Examples and Test Examples.

### Example 1 Method for synthesizing wet crystal of compound (2)

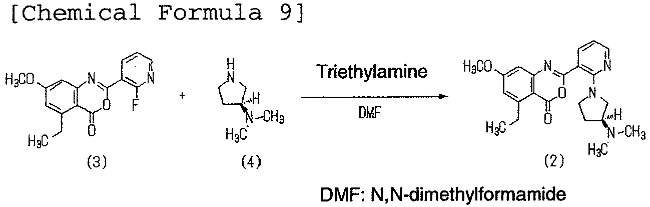

N,N-Dimethylformamide (65 mL) and triethylamine (11.1 mL) were added to the compound (3) (10.0 g) to prepare a suspension. The compound (4) (5.14 g) was added dropwise to the suspension under cooling while stirring. The container was washed with N,N-dimethylformamide (5 mL), then the washing was combined. The mixture was stirred at an inner temperature of 8 to 17°C for 2 hours, and was allowed to stand overnight at room temperature to obtain a uniform reaction solution (87.7 g).
The half amount (43.8 g) of the reaction solution was stirred under cooling. Acetone (35 mL) was added to the reaction solution and the inner temperature was lowered to 9°C. Water (175 mL) was slowly added dropwise to a mixed solution of the reaction solution and acetone at an inner temperature of 9 to 22°C to precipitate a crystal. After then, the mixture was stirred at an inner temperature of 7 to 13°C for 1 hour 20 minutes. The precipitated crystal was collected by filtration, and washed with a mixed solution of acetone (10 mL) and water (40 mL), to obtain a wet crystal of the compound (2) (9.55 g) as white powder and mass.

### Example 2 Method for synthesizing compound (1) through wet crystal of compound (2)

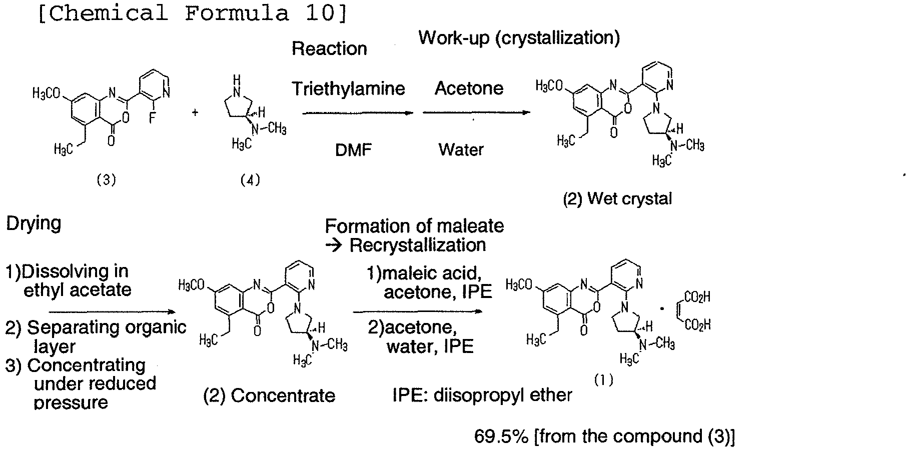

The compound (3) (10.0 g) and triethylamine (4.72 g) were suspended in N,N-dimethylformamide (65.0 mL) under stirring to prepare a suspension of the compound (3). The compound (4) (4.56 g) was added dropwise to the suspension at an inner temperature of 28 to 30°C. The container was washed with N,N-dimethylformamide (5.00 mL), then the washing was combined. The reaction solution containing the compound (3) and the compound (4) was stirred at an inner temperature of 30°C for 2 hours. The reaction solution was cooled, and acetone (90.0 mL) was added at an inner temperature of 9°C. Subsequently, water (250 mL) was slowly added dropwise to a mixed solution of the reaction solution and acetone to precipitate the compound (2) as a crystal. The mixture was stirred at an inner temperature of 8 to 13°C for 30 minutes. The precipitated crystal was collected by filtration, and washed with a mixed solution of acetone (10.0 mL) and water (100 mL), to obtain a wet crystal of the compound (2) (26.6 g) as a white mass.
The obtained wet crystal of the compound (2) (26.6 g) was dissolved in ethyl acetate (200 mL), and an organic layer was separated and concentrated under reduced pressure. Further, the obtained concentrate was dissolved by addition of ethyl acetate (200 mL), and the obtained solution was concentrated under reduced pressure to obtain a concentrate of the compound (2) (13.7 g) as a yellow oil.
The obtained concentrate of the compound (2) (13.7 g) was dissolved in acetone (230 mL) to prepare a solution of the compound (2). A solution of maleic acid (3.87 g) in acetone (50.0 mL) was added dropwise to the solution at an inner temperature of 36 to 37°C under heating while stirring, to precipitate the compound (1) as a crystal. The container was washed with acetone (20. 0 mL), then the washing was combined. And diisopropyl ether (300 mL) was added dropwise thereto. The suspension of the compound (1) was stirred under heating at inner temperature of 45 to 46°C for 10 minutes, cooled, and then stirred at an inner temperature of 7 to 15°C for 30 minutes. The precipitated crystal was collected by filtration, and washed with a mixed solution of diisopropyl ether (40.0 mL) and acetone (40.0 mL). The obtained wet product of the crude crystal of the compound (1) (14.2 g) was dried with air flow at 50°C to obtain a crude crystal of the compound (1) (13.4 g) as white powder and mass.
Acetone (200 mL) and purified water (10.0 mL) were added to the obtained crude crystal of the compound (1) (10.0 g), and the mixture was stirred under heating to dissolve the crystal. The obtained solution of the compound (1) was filtered. The container was washed with a mixed solution of acetone (30.0 mL) and purified water (1.50 mL), then the washing was combined. And the filtrate was stirred under heating. Diisopropyl ether (250 mL) was added dropwise to the filtrate at an inner temperature of 43 to 45°C to precipitate the compound (1) as a crystal. The mixture was stirred at inner temperature of 43 to 46°C for 10 minutes, cooled, and then stirred at an inner temperature of 6 to 15°C for 30 minutes. The precipitated crystal was collected by filtration, and washed with a mixed solution of diisopropyl ether (40.0 mL) and acetone (40.0 mL) . The obtained wet crystal of the compound (1) (9.28 g) was dried with air flow at 50°C to obtain the compound (1) (8.80 g) at a yield of 69.5% as white powder and mass.
Melting point (hot plate method): 157 to 163°C
Elemental Analysis Calcd. for C₂₂H₂₆N₄O₃·C₄H₄O₄ (MW: 510.54): C, 61.17%; H, 5.92%; N, 10.97%. Found: C, 61.09%; H, 5.91%; N, 10.95%.
ESI (posi) -MS m/z: 395 [(C₂₂H₂₆N₄O₃ + H)⁺].
¹H-NMR (400 MHz, DMSO-d₆) δ: 1.22 (3H, t, J = 7.6 Hz), 2.02-2.12 (1H, m), 2.29-2.36 (1H, m), 2.78 (6H, s), 3.07-3.20 (2H, m), 3.44-3.53 (2H, m), 3.68 (1H, dd, J = 11.5 Hz, 7.3 Hz), 3.76 (1H, dd, J = 11.5 Hz, 6.8 Hz), 3.83-3.96 (1H, m), 3.92 (3H, s), 6.03 (2H, s), 6.91 (1H, dd, J = 7.8 Hz, 4.9 Hz), 7.02 (1H, d, J = 2.4 Hz), 7.04 (1H, d, J = 2.4 Hz), 8.05 (1H, dd, J = 7.6 Hz, 2.0 Hz), 8.34 (1H, dd, J = 4.6 Hz, 1.7 Hz).

### Example 3 Method for synthesizing compound (1) using recrystallized purified product of compound (2)

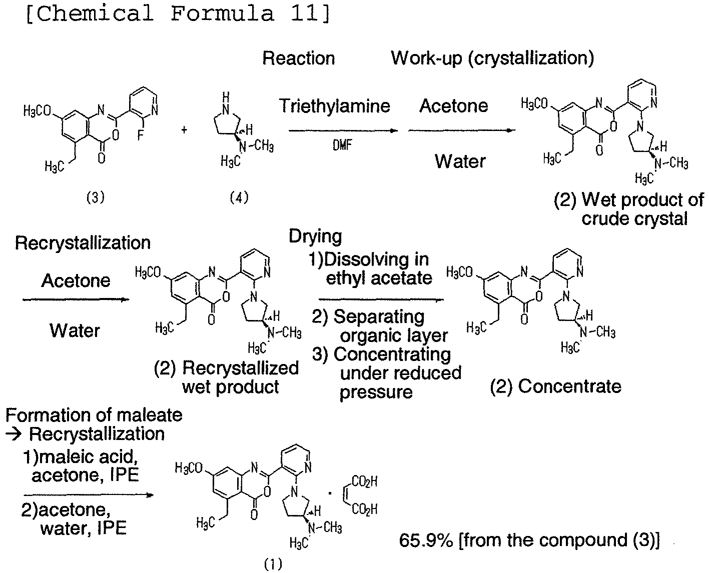

The compound (3) (10.0 g) and triethylamine (4.72 g) were suspended in N,N-dimethylformamide (65.0 mL) under stirring to prepare a suspension of the compound (3). The compound (4) (4.56 g) was added dropwise to the suspension at an inner temperature of 28 to 31°C. The container was washed with N,N-dimethylformamide (5.00 mL), then the washing was combined. The reaction solution containing the compound (3) and the compound (4) was stirred at an inner temperature of 30°C for 2 hours. The reaction solution was cooled, and acetone (90.0 mL) was added at an inner temperature of 7 to 8°C. Subsequently, water (250 mL) was slowly added dropwise to precipitate the compound (2) as a crystal. The mixture was stirred at an inner temperature 7 to 10°C for 30 minutes. The precipitated crystal was collected by filtration, and washed with a mixed solution of acetone (10.0 mL) and water (100 mL), to obtain a wet product of a crude crystal of the compound (2) (25.8 g) as a white mass.
Acetone (200 mL) was added to the obtained wet product of crude crystal of the compound (2) (25.8 g) under stirring to dissolve the crystal, and the mixture was cooled. Water (400 mL) was slowly added dropwise to the obtained solution of the compound (2) in acetone at an inner temperature of 8 to 13 °C to precipitate the compound (2) as a crystal. Then, the mixture was stirred at an inner temperature 8 to 9°C for 30 minutes. The precipitated crystal was collected by filtration, and washed with a mixed solution of acetone (10.0 mL) and water (100 mL), to obtain a recrystallized wet crystal of the compound (2) (26.1 g) as a white mass.
The obtained recrystallized wet crystal of the compound (2) (26.1 g) was dissolved in ethyl acetate (200 mL), and an organic layer was separated and concentrated under reduced pressure. Further, ethyl acetate (200 mL) was added to the obtained concentrate to dissolve it, and then the solution was concentrated under reduced pressure to obtain a concentrate of the compound (2) (12.5 g) as a yellow oil.
The obtained concentrate of the compound (2) (12.5 g) was dissolved in acetone (230 mL) to prepare the solution of the compound (2). A solution of maleic acid (3.87 g) in acetone (50.0 mL) was added dropwise to the resulting solution under heating while stirring at an inner temperature of 36°C to precipitate the compound (1) as a crystal. The container was washed with acetone (20.0 mL), then the washing was combined. And diisopropyl ether (300 mL) was added dropwise thereto. The suspension of the compound (1) was stirred under heating at inner temperature of 45 to 47°C for 10 minutes, cooled, and then stirred at an inner temperature of 8 to 15°C for 30 minutes. The precipitated crystal was collected by filtration, and washed with a mixed solution of diisopropyl ether (40.0 mL) and acetone (40.0 mL). The obtained wet product of the crude crystal of the compound (1) (15.2 g) was dried with air flow at 50°C to obtain a crude crystal of the compound (1) (12.7 g) as white powder and mass.
Acetone (200 mL) and purified water (10.0 mL) were added to the obtained crude crystal of the compound (1) (10.0 g) under heating while stirring to dissolve the crystal. The obtained solution of the compound (1) was filtered. The container was washed with a mixed solution of acetone (30.0 mL) and purified water (1.50 mL), then the washing was combined, and the filtrate was stirred under heating. Diisopropyl ether (250 mL) was added dropwise to the filtrate at an inner temperature of 43 to 45°C to precipitate the compound (1) as a crystal. Then, the mixture was stirred at inner temperature of 44 to 45°C for 10 minutes, cooled, and then stirred at an inner temperature of 6 to 15°C for 30 minutes. The precipitated crystal was collected by filtration, and washed with a mixed solution of diisopropyl ether (40.0 mL) and acetone (40.0 mL). Then the obtained wet crystal of the compound (1) (9.31 g) was dried with air flow at 50°C to obtain the compound (1) (8.83 g) at a yield of 65.9% as white powder and mass.
Melting point (hot plate method): 164 to 165°C
Elemental Analysis Calcd. for C₂₂H₂₆N₄O₃·C₄H₄O₄ (MW: 510.54): C, 61.17%; H, 5.92%; N, 10.97%. Found: C, 61.07%; H, 5.87%; N, 10.96%.
ESI(posi)-MS m/z: 395 [(C₂₂H₂₆N₄O₃ + H)⁺].
¹H-NMR (400 MHz, DMSO-d₆) δ: 1.22 (3H, t, J = 7.3 Hz), 2.02-2.12 (1H, m), 2.29-2.36 (1H, m), 2.78 (6H, s), 3.09-3.18 (2H, m), 3.45-3.52 (2H, m), 3.68 (1H, dd, J = 11.7 Hz, 7.3 Hz), 3.76 (1H, dd, J = 11.7 Hz, 7.1 Hz), 3.83-3.95 (1H, m), 3.92 (3H, s), 6.03 (2H, s), 6.91 (1H, dd, J = 7.6 Hz, 4.6 Hz), 7.02 (1H, d, J = 2.7 Hz), 7.04 (1H, d, J = 2.7 Hz), 8.05 (1H, dd, J = 7.8 Hz, 2.0 Hz), 8.34 (1H, dd, J = 4.6 Hz, 2.0 Hz).

### Example 4 Method for producing wet crystal of compound (2) from compound (1)

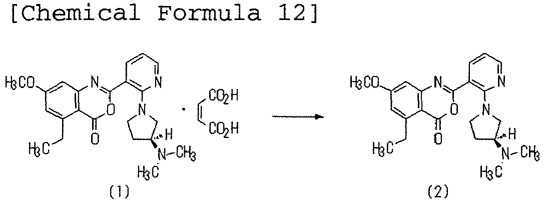

Ethyl acetate (300 mL) and a solution of sodium bicarbonate (20.0 g) and water (300 mL) were sequentially added to the crystal of the compound (1) described in PCT/JP2010/005786 (20.0 g, a mixture of a high melting point crystal and a low melting point crystal). The compound (1) was dissolved at an inner temperature of 16 to 17°C under stirring. The solution of the compound (1) was stirred at an inner temperature of 13 to 18°C for 20 minutes to prepare the compound (2). Then the organic layer containing the compound (2) was separated, and washed with a solution of sodium bicarbonate (6.00 g) and water (100 mL). Anhydrous sodium sulfate (30.0 g) was added to the obtained organic layer. The mixture was stirred and allowed to stand overnight to dry the solution of the compound (2). An insoluble material was removed by filtration, and washed with ethyl acetate (20.0 mL). The washing was combined with the filtrate. The filtrate was concentrated under reduced pressure to obtain the compound (2) (17.7 g) as a yellow oil.
Acetone (177 mL) was added to the obtained compound (2) (17.7 g) to dissolve the compound. Water (472 mL) was slowly added dropwise to the solution of the compound (2) at an inner temperature of 17 to 33 °C under stirring to precipitate the compound (2) as a crystal. Then the mixture was stirred under cooling at an inner temperature 8 to 15°C for 30 minutes. The resulting crystal was collected by filtration, and washed with a mixed solution of acetone (11.8 mL) and water (59.0 mL), to obtain a wet crystal of the compound (2) (32.7 g) as a yellowish white mass.

### Reference Example 1 Method for drying wet crystal of compound (2)

The wet crystal of the compound (2) obtained in Example 4 was dried at room temperature under reduced pressure to obtain the dried compound (2) (14.6 g) at a yield of 93.4% as a pale yellow powder.
Melting point (hot plate method) : glass transition occurred at 48°C
Water content (Karl Fischer): 1.32%
¹H-NMR (CDCl₃) δ: 1.30 (3H, t, J = 7.6 Hz), 1.80-1.88 (1H, m), 2.08-2.15 (1H, m), 2.25 (6H, s), 2.72-2.80 (1H, m), 3.15-3.28 (2H, m), 3.43-3.56 (3H, m), 3.66 (1H, dt, J = 6.6 Hz, 10.7 Hz), 3.92 (3H, s), 6.72 (1H, dd, J = 7.8 Hz, 4.9 Hz), 6.88 (1H, d, J = 2.7 Hz), 6.95 (1H, d, J = 2.7 Hz), 8.00 (1H, dd, J = 7.8 Hz, 2.0 Hz), 8.30 (1H, dd, J = 4.9 Hz, 2.0 Hz).
ESI-MS(posi) m/z: 395 (M+1)⁺
Elemental Analysis Calcd. for C₂₂H₂₆N₄O₃: C, 66.99; H, 6.64; N, 14.20. Found: C, 65.89; H ,6.66; N, 13.96.

### Test Example 1 X-ray diffraction of wet crystal of compound (2)

FIG. 1 shows a result of powder x-ray diffraction of wet crystal of the compound (2) (Example 1). From the result of powder x-ray diffraction, the wet crystal is confirmed to be a crystalline powder and a mass. The conditions of powder x-ray diffraction measurement are as follows.
Powder x-ray diffraction:
A filling section of a plate sample holder made of glass was filled with a sample, and powder x-ray diffraction of the sample was then measured using RINT2200 powder x-ray diffractometer manufactured by Rigaku Corporation.
Measurement Conditions
Tube current: 36 mA
Tube voltage: 40 kV
Scanning rate: 2°/min
wavelength: 1.5405 Å (Kα1), 1.5443 Å (Kα2), 1.3922 Å (Kβ1)
Divergence slit: 1°
Receiving slit: 0.15 mm
Scattering slit: 1°
Anticathode: copper
Scanning range: 5 to 40°

### Test Example 2 Powder x-ray diffraction of dried wet crystal of compound (2)

A plate sample holder used in Test Example 1 was filled with the wet crystal of the compound (2) (Example 1). The wet crystal was dried in a silica gel (blue) desiccator to obtain a dried product. When powder x-ray diffraction was measured in accordance with the method of Test Example 1, a diffraction pattern of amorphous form was observed as shown in FIG. 2.

### Test Example 3 Stability test of compound (2) (powder x-ray diffraction)

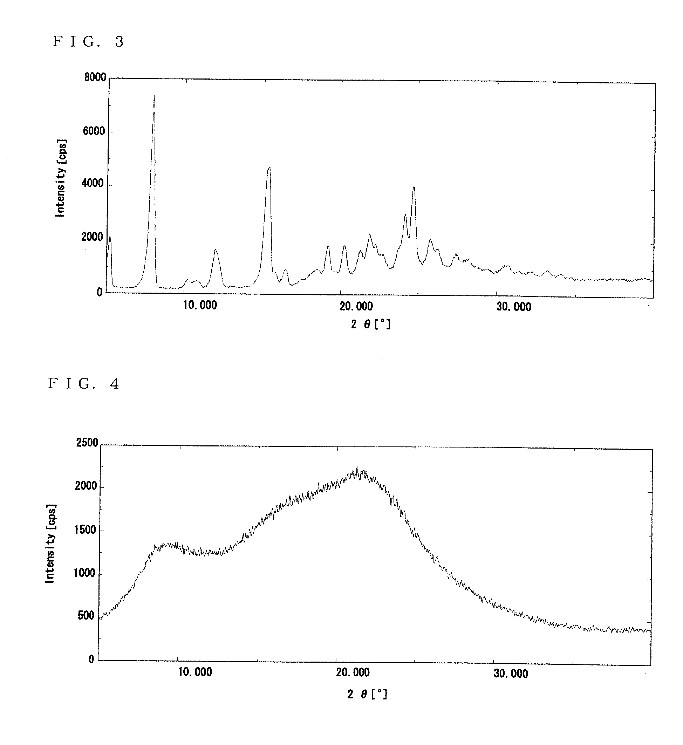

The wet crystal of the compound (2) (Example 1) and the dried product (Reference Example 1) were each sealed in a brown bottle, and stored at 25°C for 2 weeks and 4 weeks. After the storage, the powder x-ray diffraction of each sample was measured in accordance with the method of Test Example 1. The results are shown in FIGs. 3 and 4. The dried product of the compound (2) was in a complete amorphous form. On the other hand, change in the crystal state of the wet crystal of the compound (2) was not observed.

### Test Example 4 Stability test of compound (2) (physical properties)

The wet crystal of the compound (2) (Example 1) and the dried product (Reference Example 1) were each sealed in a brown bottle, and stored at 25 to 40°C for 2 weeks and 4 weeks. After the storage, the physical properties of each sample were checked. On 3 days after the storage at 30°C, the dried product was changed into a glassy mass.
Physical properties of dried product of compound (2) (Reference Example 1)

**[Table 1]**

| AT THE START | 4 WEEKS (25°C) | 4 WEEKS (30°C) |
|---|---|---|
| YELLOWISH WHITE POWDER AND MASS | YELLOWISH WHITE POWDER AND MASS | CHANGED INTO GLASSY MASS |

### Physical properties of wet crystal of compound (2) (Example 1)

**[Table 2]**

| AT THE START | 2 WEEKS (25°C) | 2 WEEKS (30°C) |
|---|---|---|
| PALE YELLOW WHITE POWDER AND MASS | WHITE POWDER AND MASS | WHITE POWDER AND MASS |

When the dried product of the compound (2) was stored under a condition of 30°C, it was changed into a glassy solid, which is difficult to be handled. On the other hand, when the wet crystal was stored under a condition of 30°C, the state of white powder was maintained. Therefore, transportation, weighing, and preparation for reaction after storage are easy to the wet crystal.

### Test Example 5

The chemical purities of the compounds (2) and (1) produced in Examples 2 and 3, respectively, were measured under the following HPLC conditions. HPLC measurement method:
HPLC column: Inertsil ODS-3V (4.6 mm ID x 150 mm, particle diameter: 5 µm)
Measurement wavelength: 240 nm
Column temperature: 35°C
Flow rate: 1.0 mL/min
Mobile phase A: liquid in which sodium 1-octanesulfonate (1. 08
g) is dissolved in diluted phosphoric acid (1→1,000) into a volume of 1,000 mL
This diluted phosphoric acid (1→1,000) means that 1 mL of phosphoric acid is dissolved in water to 1,000 mL.
Mobile phase B: acetonitrile for liquid chromatography Mobile phase condition

**[Table 3]**

| TIME AFTER INJECTION (MIN) | MOBILE PHASE A (%) | MOBILE PHASE B (%) |
|---|---|---|
| 0∼18 | 70 | 30 |
| 18∼30 | 70→60 | 30→40 |
| 30∼50 | 60→30 | 40→70 |
| 50∼55 | 30 | 70 |

In all Examples, the chemical purities of the obtained compounds (2) and (1) were confirmed to be 99% or more.

### Industrial Applicability

The present invention can provide a maleate excellent in storage stability, solubility, crystallinity, and easy of handling at high purity and efficiency (without column purification).

## Claims

1. A method for producing a crystal of a maleate represented by the formula (1), comprising
reacting a compound represented by the formula (3) with a compound represented by the formula (4) in the presence of a base to obtain a compound represented by the formula (2);
mixing the obtained compound represented by the formula (2) with acetone and water to precipitate a crystal of the compound represented by the formula (2); and
adding maleic acid to a solution of the compound represented by the formula (2) prepared by dissolving, in a solvent, the obtained crystal of the compound represented by the formula (2) directly or after being dried to obtain a crystal of a maleate represented by the formula (1).

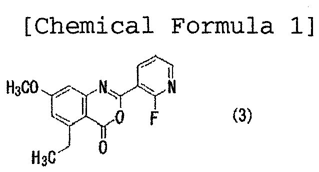

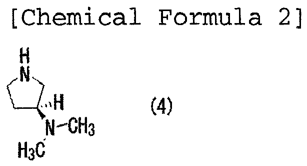

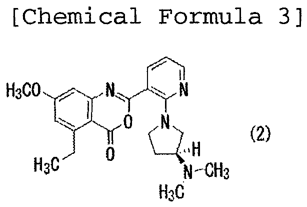

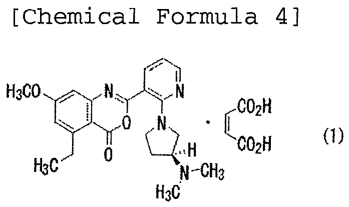

2. The method for producing a crystal of a maleate represented by the formula (1) according to claim 1, wherein the crystal of the compound represented by the formula (2) is dried and then dissolved in the solvent to prepare the solution of the compound represented by the formula (2), and
the crystal of the compound represented by the formula (2) is dried under reduced pressure, or dried by dissolving the crystal in a solvent capable of separating from water into two layers, removing separated water, and concentrating the solution.

3. A crystal of a compound represented by the formula (2) having a particular peak at a diffraction angle (2θ ± 0.5°) of 15.4° in a powder X-ray diffraction.

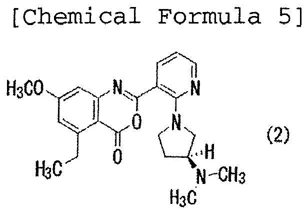

4. A crystal obtained by mixing a compound represented by the formula (2) with acetone and water to precipitate the crystal.

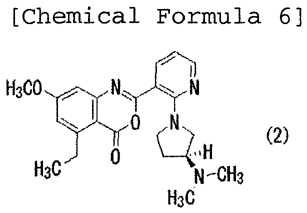

5. A method for producing a crystal of a maleate represented by the formula (1), comprising
dissolving the crystal described in claim 2 or 3 directly or after being dried in a solvent to prepare a solution of a compound represented by the formula (2); and
adding maleic acid to the obtained solution of the compound represented by the formula (2) to obtain a crystal of a maleate represented by the formula (1).

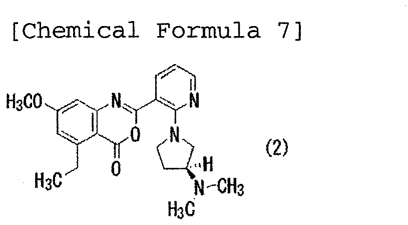

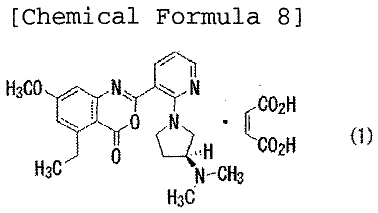

6. The method for producing a crystal of a maleate represented by the formula (1) according to claim 5, wherein the crystal of the compound represented by the formula (2) is dried and then dissolved in the solvent to prepare the solution of the compound represented by the formula (2), and
the crystal of the compound represented by the formula (2) is dried under reduced pressure or dried by dissolving the crystal in a solvent capable of separating from water into two layers, removing separated water, and concentrating the solution.
